# EUROPEAN PATENT SPECIFICATION

(11) **EP 3 192 557 B1**
(45) Date of publication and mention of the grant of the patent: **12.05.2021**
(21) Application number: 15840118.2
(22) Date of filing: 08.09.2015
(51) Int. Cl.: A61M 37/00

(54) **PUNCTURE DEVICE**
PUNKTIONSVORRICHTUNG
DISPOSITIF DE PIQÛRE

(30) Priority: 08.09.2014 JP 2014182572; 07.09.2015 JP 2015175878
(43) Date of publication of application: 19.07.2017
(73) Proprietor: Kaiwa Co., Ltd., Uenohara-shi, Yamanashi 409-0112 (JP)
(72) Inventor: YAMAZOE, Shigeyuki, Uenohara-shi Yamanashi 409-0112 (JP)
(74) Representative: Strehl Schübel-Hopf & Partner
(86) International application number: PCT/JP2015/075453
(87) International publication number: WO 2016/039333

(56) References cited:
- EP-A1- 1 109 594
- EP-A1- 2 036 586
- EP-A1- 2 578 264
- WO-A2-02/062202
- WO-A2-2005/049107
- WO-A2-2009/014805
- DE-A1-102008 052 702
- DE-A1-102008 052 749
- JP-A- 2000 512 529
- JP-A- 2003 093 521
- JP-A- 2004 516 868
- JP-A- 2004 526 581
- JP-A- 2008 528 192
- JP-A- 2009 502 261
- JP-A- 2009 535 122
- JP-A- 2012 532 709
- JP-A- 2013 513 408
- JP-A- 2014 519 385
- US-A- 5 457 041
- US-A1- 2012 016 309
- US-A1- 2013 331 792

## Description

### [Technical Field]

The present invention relates to a puncture device, and more particularly to a puncture device used for percutaneous administration of a drug.

### [Background Art]

As a puncture device, a microneedle device has been disclosed, which includes a plurality of microneedles for puncturing the skin and allows noninvasive administration of a drug. The microneedle device punctures a horny layer, which is the outermost layer of the skin, with the microneedles to percutaneously administer a drug. Various sizes and shapes of microneedles have been proposed to promote the percutaneous absorption of a drug (for example, patent document 1). Patent document 1 discloses microneedles having a conical shape and a knife-shape. The document also discloses that microneedles can be formed from metal, ceramic, or plastic.
The document DE 10 2008 052749A1 discloses microneedle arrays wherein a microneedle is disposed on a carrier. Protrusions which have strongly curved cutting edges are provided on the end of a needle. The microneedle has an elongated needle body.
The document US 2013/0331792 A1 discloses "oval" protrusions adjacent to a tip configured to spread the tissue. The document WO 2005/049107 A2 discloses a tubular microneedle with a substantially elliptical cross-section but without a cutting edge extending along the middle line. The document WO 00/12173) discloses various shapes of blade portions. The side faces of the blade portions are flat.
Further related art is disclosed in WO 02/062202 A2, US 2013/331792 A1, EP 2 578 264 A1 and EP 2 036 586A1.

### [Citation List]

### [Patent Document]

Patent Document 1: Japanese Translation of PCT International Application Publication No. 2010-502268

### [Summary of the Invention]

### [Problems to be Solved by the Invention]

The microneedle disclosed in the patent document 1 has a fine distal end. When, for example, such a microneedle is formed from plastic, the distal end deforms as it is pressed against the skin. This leads to a failure in puncturing the skin to an extent that allows the administration of a drug.

An object of the present invention is to provide a puncture device which punctures the skin more reliably.

### [Means for Solving the Problems]

To solve the above problems, the invention proposes a puncture device as defined in claim 1. A puncture device according to the present invention includes a basal portion and a plurality of blade portions disposed on the basal portion. The blade portion has a thin shape, and its distal end portion has a convex shape.

### [Advantageous Effects of Invention]

According to the present invention, the blade portion has a thin shape and hence does not easily deform as compared with a needle-shape. Since the distal end portion of the blade portion has a convex shape, the blade portion easily goes into the skin. This allows the blade portion to cut the skin when a force is applied to the blade portion in one direction by moving the tooth of the needle in its longitudinal direction while the blade portion is pressed against the skin. Therefore, with the blade portions, which cut the skin, the puncture device more reliably punctures the skin.

### [Brief Description of the Drawings]

Fig. 1 is a perspective view showing an overall configuration of a puncture device.
Figs. 2A to 2C show a configuration of a blade portion according to a first
   example, not forming part of the invention:

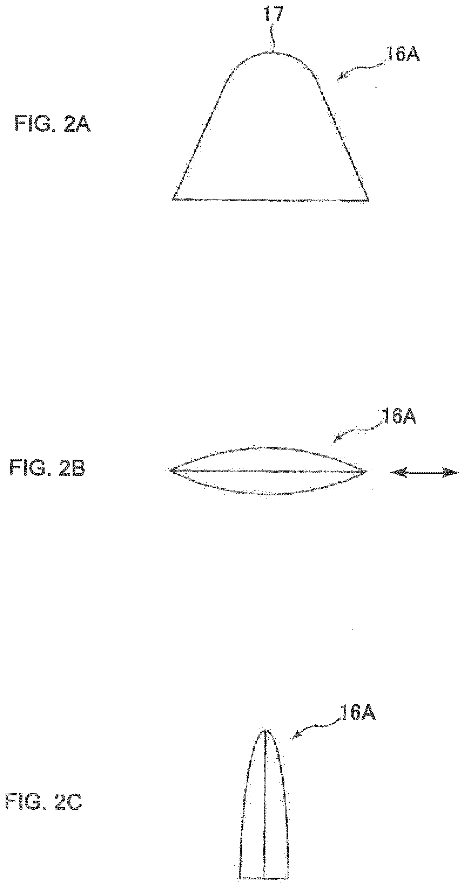

Fig. 2A is a front view; Fig. 2B is a plan view; and Fig. 2C is a side view

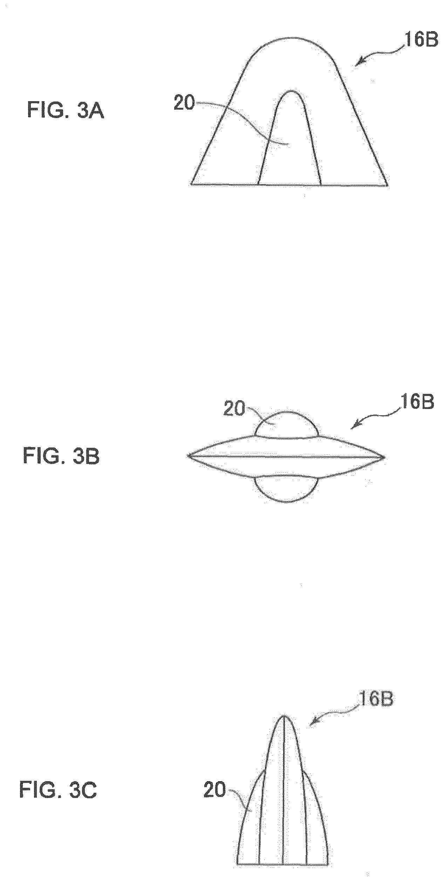

Figs. 3A to 3C show a configuration of a blade portion according to a second example, not forming part of the invention:
Fig. 3A is a front view; Fig. 3B is a plan view; and Fig. 3C is a side view

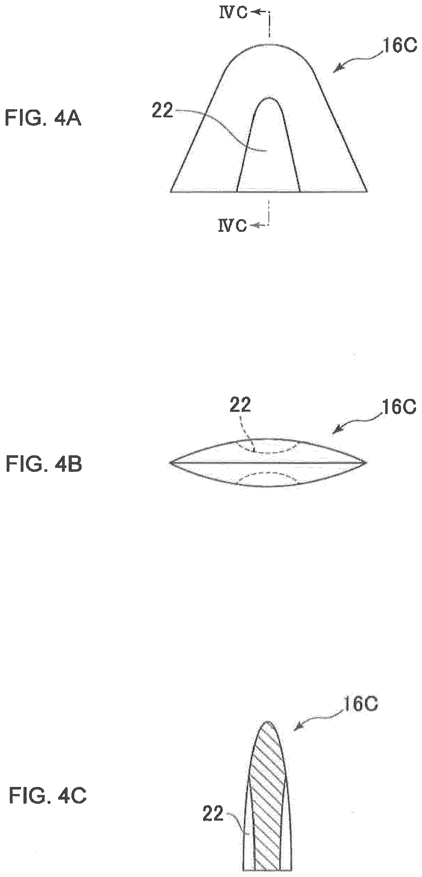

Figs. 4A to 4C show a configuration of a blade portion according to a modification of the second example:
Fig. 4A is a front view; Fig. 4B is a plan view; and Fig. 4C is a cross-sectional view cut along a line IVC-IVC in Fig. 4A.

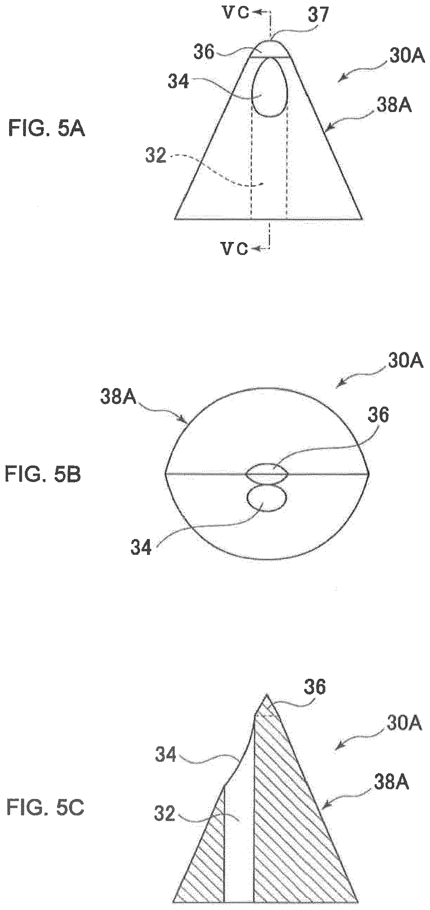

Figs. 5A to 5C show a configuration of a blade portion according to a first embodiment of the invention:
Fig. 5A is a front view; Fig. 5B is a plan view; and Fig. 5C is a cross-sectional view cut along a line VC-VC in Fig. 5A;

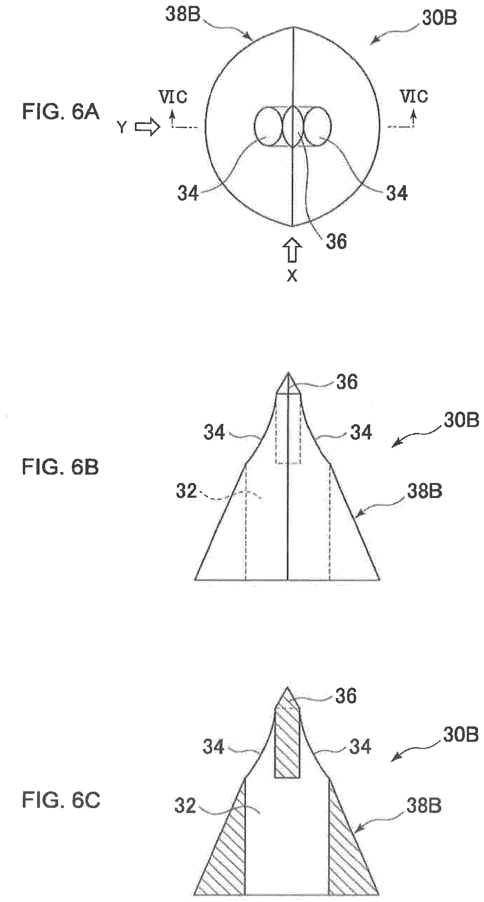

Figs. 6A to 6C show a configuration of a blade portion according to a modification of the first
   embodiment: Fig. 6A is a plane view; Fig. 6B is a view taken along an arrow X; and Fig. 6C is a cross-sectional view cut along a line VIC-VIC in Fig. 6A;

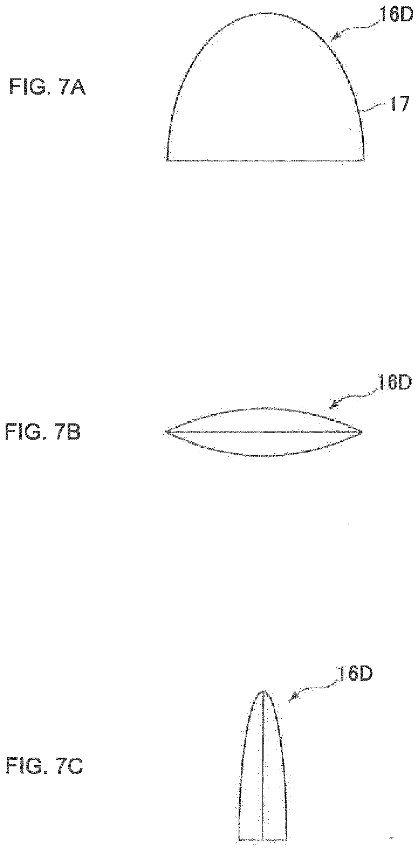

Figs. 7A to 7C show a configuration of a blade portion according to modified first example, which does not form part of the invention:
   Fig. 7A is a front view; Fig. 7B is a plan view; and Fig. 7C is a side view;

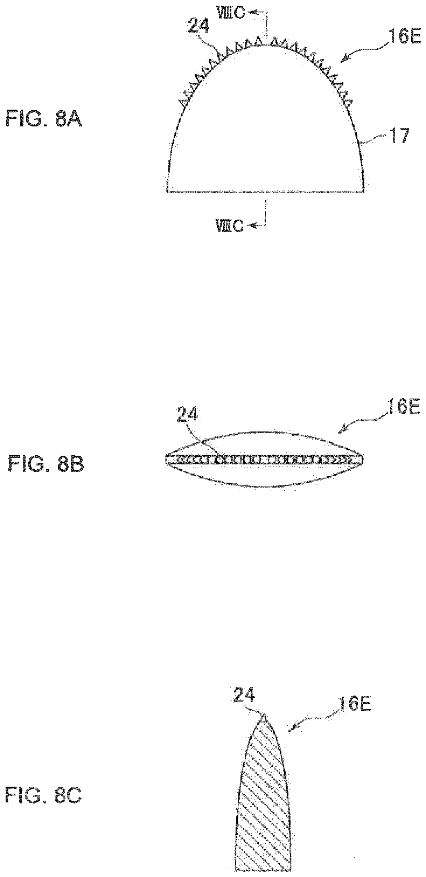

Figs. 8A to 8C show a configuration of a blade portion according to modified second example, which does not form part of the invention: Fig. 8A is a front view; Fig. 8B is a plan view; and Fig. 8C is a cross-sectional view cut along a line VIIIC-VIIIC in Fig. 8A.

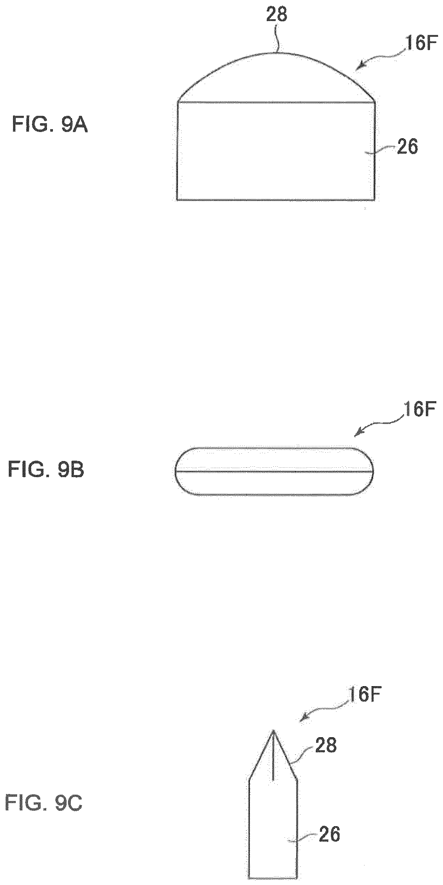

Figs. 9A to 9C show a configuration of a blade portion according to modified first example;

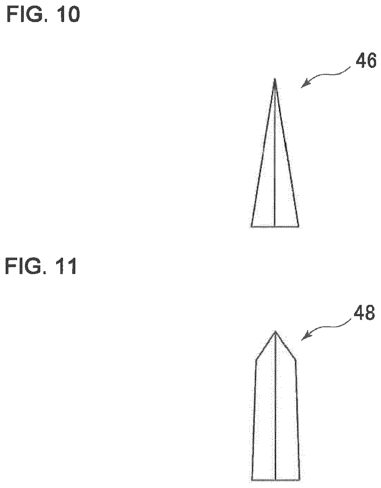

Fig. 9A is a front view; Fig. 9B is a plan view; and Fig. 9C is a side view Fig. 10 is a side view showing a configuration of a blade portion according to modified embodiment (2); and
Fig. 11 is a side view showing a configuration of a blade portion according to modified embodiment (3).

### [Description of the Examples and Embodiments]

### [Examples]

Examples and Embodiments of the present invention and related to the invention will be described in detail below with reference to the accompanying drawings.

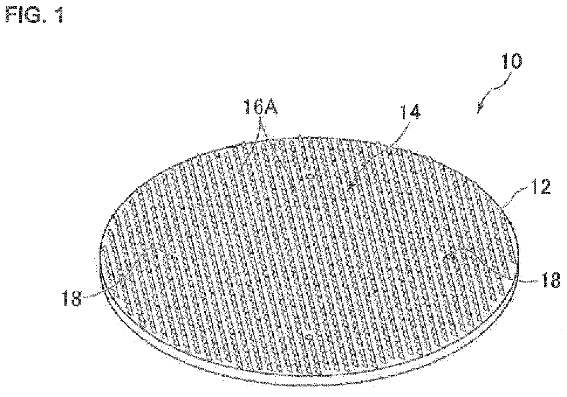

1. First Example not forming part of the invention A puncture device 10 shown in Fig. 1 includes a basal portion 12 and a puncture portion 14. The puncture device 10 is formed from a synthetic resin, for example, a thermoplastic resin. The basal portion 12 shown in Fig. 1 has a discoid shape. The puncture portion 14 is disposed on one (first surface) of two surfaces of the basal portion 12.

The puncture device 10 may include channels 18, which guide a drug. In this example, the channel 18 is a hole penetrating the basal portion 12 in the thickness direction of the basal portion 12.

The puncture portion 14 includes a plurality of blade portions 16A. A metal film may be formed on the surface of the puncture portion 14 to improve its mechanical strength.

As shown in Figs. 2A to 2C, the blade portion 16A has a thin shape, and its distal end portion 17 has a convex shape when viewed from the front. In this example, when viewed from the front, the distal end portion 17 has a gentle curve, and the blade portion 16A gradually extends in the widthwise direction toward the proximal end, and thus, the blade portion 16A as a whole has a convex shape like a shark tooth. The blade portion 16A has a substantially elliptical shape when viewed from above. When viewed from the side, the proximal end of the blade portion 16A has a certain thickness, and the blade portion 16A tapers from the proximal end to the distal end. When viewed from the side, the blade portion 16A shown in Fig. 2C has a shape similar to the sides of shells of a clam.

The blade portions 16A having the above-described configuration are arranged on the basal portion 12 with the distal end portions 17 aligned in the same direction (that is, along an arrow direction in Fig. 2B). Thus, the blade portions 16A constitute the puncture portion 14 (Fig. 1).

The puncture device 10 is produced by typical injection molding. A mold (not shown) is preferably formed by combining a plurality of molds in accordance with the shape of the puncture device 10. For example, the blade portion 16A is formed by a mold that can be divided into two parts in the thickness direction. A molten thermoplastic resin is injected into the mold and held by the mold for a predetermined period of time. Thereafter, the mold is opened. Thus, the puncture device 10 is produced.

The other surface (second surface) of the basal portion 12 of the puncture device 10 having the above configuration is attached to a device (not shown) which holds a drug. The puncture portion 14 is brought into contact with the skin and slid in one direction, e.g. a lateral direction (the arrow direction in Fig. 2B) while the puncture portion 14 is pressed against the skin. Thus, the puncture portion 14 punctures a horny layer of the skin. Thereby, the drug guided from the second surface to the first surface of the basal portion 12 through the channels 18 in the basal portion 12 is percutaneously administered through cuts in the skin cut by the puncture portion 14.

In this example, the blade portion 16A included in the puncture portion 14 has a thin shape, and the distal end portion 17 has a convex shape when viewed from the front. Hence the blade portion 16A does not easily deform as compared with a conventional needle-shape, and easily goes into the skin. This allows the blade portion 16A to cut the skin when a force is applied to the blade portion in one direction while the blade portion 16A is pressed against the skin. Therefore, with the puncture portion 14 including the plurality of blade portions 16A, which cut the skin, the puncture device 10 punctures the skin more reliably.

When viewed from the side, the proximal end of the blade portions 16A has the certain thickness and the blade portion 16A tapers from the proximal end to the distal end. This further suppresses the deformation of the distal end when the blade portion 16A is pressed against the skin.

2. Second Example, not forming part of the invention Blade portions according to a second example will be described next. The blade portion according to this example differs from the blade portion according to the first example in that a drug permeation portion is formed. The blade portion according to the second example will be described with reference to Figs. 3A to 3C, with the same reference numerals denoting the same components as in Figs. 2A to 2C.

A blade portion 16B shown in Figs. 3A to 3C has a thin shape, and includes the distal end portion 17 having a convex shape, when viewed from the front, and widened portions 20. The widened portion 20 corresponds to a drug permeation portion. The widened portion 20 includes a protrusion extending linearly from the proximal end of the blade portion 16B toward the distal end of the blade portion 16B. The widened portion 20 is disposed approximately in the middle, when viewed from the front, of each of first and second surfaces of the blade portion 16B.

The blade portion 16B has the thin shape, and the distal end portion 17 has the convex shape when viewed from the front. Hence, effects similar to those of the first example are achieved. In addition, when the blade portion 16B cuts the skin, the widened portions 20 widen the cut made in the skin. A drug permeates the skin through the cut widened by the blade portion 16B and the widened portions 20. Thereby, a drug is administered more efficiently.

The drug permeation portion is not limited to the widened portion 20 and may include a groove 22 as shown in, for example, Figs. 4A to 4C. As shown in Figs. 4A to 4C, the groove 22 extends linearly from the proximal end of a blade portion 16C toward the distal end the blade portion 16C. The groove 22 is disposed approximately in the middle, when viewed from the front, of each of first and second surfaces of the blade portion 16C. When the blade portion 16C cuts the skin, the groove 22 forms a gap between the cut of the skin and the blade portion 16C. A drug permeates the skin through the gap. Thereby, the drug is administered more efficiently.

Channels may be formed through the basal portion at the proximal ends of the grooves 22 of the blade portion 16C. The channel is preferably a hole extending through the basal portion in the thickness direction. Hence, when the blade portion 16C cuts the skin, the drug, which is guided from the second surface to the first surface of the basal portion through the channel, is guided through the groove 22 to the cut in the skin. Thereby, the drug is administered more efficiently.

3. First Embodiment of the invention In the embodiment of the invention, the blade portion corresponds to a blade edge of a bladed drug supply member including a support portion integrally provided with the proximal end of the blade portion. A plurality of bladed drug supply members constitute a puncture portion of a puncture device. In this puncture device, the blade portion is fixed to the basal portion through the support portion. The bladed drug supply member having the blade portion, being the blade edge, will be described below with reference to Figs. 5A to 5C.

The puncture device is attached to a syringe or the like and used like an injector, to supply a drug solution. The puncture device has a through hole, which supplies a drug solution. As shown in Figs. 5A to 5C, a bladed drug supply member 30A has a support portion 38A integrally provided with the proximal end of a blade portion 36. The blade portion 36, being the blade edge, of the bladed drug supply member 30A cuts the skin to make a cut.

The blade portion 36 has substantially the same configuration as that of the blade portion 16A according to the first example. The blade portion 36 has a thin shape, and a distal end portion 37 has a convex shape when viewed from the front. The blade portion 36 has a substantially elliptical shape when viewed from above. When viewed from the front, the distal end portion 37 has a gentle curve, and the blade portion 36 gradually extends in the widthwise direction toward the proximal end, and the blade portion 36 as a whole has a convex shape like a shark tooth. In this regard, the blade portion 36 is substantially the same as the blade portion 16A according to the first example. In the first embodiment, however, the blade portion 36 constitutes the blade edge of the bladed drug supply member 30A, and hence is smaller in size than the blade portion 16A (Figs. 2A to 2C) in the first example. The bladed drug supply member 30A, as a whole, may have the same shape and the same size as the blade portion 16A when viewed from the front. As will be described below, since the support portion 38A is required to have a certain thickness, the bladed drug supply member 30A is thicker than the blade portion 16A.

The support portion 38A, which is disposed between the basal portion (not shown) and the blade portion 36, supports the blade portion 36. As will be described below, the support portion 38A supplies the drug, which is guided through the basal portion, to the blade portion 36 or the cut. In this embodiment, the support portion 38A has an isosceles trapezoidal shape when viewed from the front. Since the support portion 38A is integrally provided with the proximal end of the blade portion 36, the upper end of the support portion 38A coincides with the proximal end of the blade portion 36. A through hole 32 is provided inside the support portion 38A, and hence the support portion 38A is required to have a certain thickness. In this embodiment, the support portion 38A has a substantially circular-shaped bottom surface when viewed from above.

The through hole 32 is formed inside the support portion 38A to supply a drug from the channel of the basal portion to the blade portion 36. One end of the through hole 32 communicates with the channel. The other end of the through hole 32 reaches the blade portion 36. Hence, the drug is directly guided to the blade portion 36 from the second surface of the basal portion through the channel of the basal portion and the through hole 32 of the support portion 38A. The transverse sectional shape of the through hole 32 is not specifically limited. In this embodiment, the transverse sectional shape of the through hole 32 is approximately circular.

The support portion 38A is not limited to any specific shape and may take any shape as long as the blade portion 36 has the thin shape and the blade portion 36 including the convex-shaped distal end portion 37 is integrally provided with the upper end of the support portion 38A and the through hole 32, which guides a drug to the blade portion 36, is formed inside the support portion 38A. The bladed drug supply members 30A constitute the puncture portion of the puncture device. Each of the bladed drug supply members 30A includes the support portion 38A, which is integrally provided with the lower end of the blade portion 36, and the blade portion 36, which corresponds to the blade edge.

The puncture device including the plurality of bladed drug supply members 30A as the puncture portion is produced by typical injection molding as in the first example. A mold (not shown) is preferably formed by combining a plurality of molds so as to produce the support portion 38A integrally with the blade portion 36 as described above. For example, the bladed drug supply member 30A is formed by a mold which includes a rod for forming the through hole 32 inside the support portion 38A and which can be divided into two parts in the thickness direction. The puncture device according to this embodiment is produced in the same manner as in the first example except for the use of the predetermined mold.

In this embodiment, the blade portion 36 has a thin shape, and the distal end portion 37 has a convex shape when viewed from the front. Hence, the effects similar to those of the first example are achieved. The through hole 32 inside the support portion 38A of the bladed drug supply member 30A is provided to communicate with the channel of the basal portion (not shown). Since the support portion 38A includes the through hole 32 communicating with the channel, when the blade portion 36 cuts the skin, a drug is guided from the second surface of the basal portion into the through hole 32 of the support portion 38A through the channel. The drug is supplied from an opening end 34 of the through hole 32 to the blade portion 36, being the blade edge, and permeates the skin through the cut. Thereby, the drug is supplied close to the blade portion 36, so that the drug is administered more efficiently.

After the blade portion 36 cuts the skin, the puncture device is pushed to insert at least a part of the opening end 34 of the through hole 32 of the bladed drug supply member 30A into the cut. Thereby, the drug is administered. In this case, the drug guided from the second surface of the basal portion through the channel is directly supplied from the opening end 34 into the cut. Thereby, the drug is administered more efficiently.

The number of the opening end 34 of the support portion 38A is not limited to one and the support portion 38A may be provided with a plurality of opening ends. For example, as shown in Fig. 6A, the opening ends 34 may be respectively provided on the first and second surfaces of a support portion 38B. The blade portion 36 is disposed between the opening ends 34. In a bladed drug supply member 30B, the support portion 38B is integrally provided with the proximal end of the blade portion 36. Fig. 6B is a view of the bladed drug supply member 30B including the support portion 38B, taken along an arrow X. Although not shown, a view of the bladed drug supply member 30B taken along an arrow Y is similar to the front view shown in Fig. 5A. The two opening ends 34 of the bladed drug supply member 30B are the opening ends of the single through hole 32 as shown in Fig. 6C, which is the cross-sectional view cut along a line VIC-VIC. The two opening ends may be the opening ends of different through holes. The two opening ends 34 increase the amount of drug supplied to the blade portion 36 through the through hole 32. Hence, the efficiency of administration is further improved.

4. Modified examples not forming part of the invention The above examples may be changed as follows:

For example, as shown in Figs. 7A to 7C, a blade portion 16D having a substantially semicircular shape, when viewed from the front, may be used. In this case, the blade portion 16D has a thin shape, and the distal end portion 17 has a convex shape when viewed from the front. Hence, effects similar to those of the above-described first example are achieved.

As shown in Figs. 8A to 8C, a blade portion 16E may include teeth (serrations) 24 on the distal end portion 17. The blade portion 16E with the teeth 24 cuts the skin more efficiently.

As shown in Figs. 9A to 9C, a blade portion 16F may include a plate-shape proximal end portion 26 and a distal end portion 28 having a convex shape. The blade portion 16F has an oval shape when viewed from above. With the blade portion 16F having the thin shape and the distal end portion 28 having the convex shape when viewed from the front, effects similar to those of the first 2. example are achieved. The plate-shaped proximal end portion 26 improves the mechanical strength of the blade portion 16F to allow the formation of the sharper distal end portion 28. The blade portion 16F therefore cuts the skin more efficiently.

Each of the blade portions 16D to 16F according to the above modified examples may be provided with the drug permeation portion described in the second example. Each of the blade portions 16D to 16F according to the modified examples may have an appropriate size, and the support portion described in the first embodiment may be integrally provided with the lower end of the blade portion, to form a bladed drug supply member. The puncture portion may be formed by combining various types of the blade portions according to the above embodiments and the modified embodiments.

In the above embodiments and examples the blade portions have the same size. The present invention is not limited to this. The blade portions located in a center portion of the puncture portion may protrude higher in the direction toward their distal ends than those in a peripheral portion of the puncture portion . The puncture portion having such a shape is produced by making a center portion of the basal portion protrude toward the distal ends of the blade portions. Thereby, the entire puncture portion of the puncture device makes the cuts having substantially the same depth in the skin.

The above embodiments and examples describe the puncture device formed from a synthetic resin. The present invention is not limited to this. The puncture device may be formed from a polylactic acid biodegradable polymer or a metal such as stainless steel, titanium, silver, or gold. As in the case in which a synthetic resin is used, in a case where a puncture device is formed from a polylactic acid biodegradable polymer, a metal film may be provided on the surface of the blade portion to increase the mechanical strength of the blade portion. When a puncture device is formed from metals, the puncture device may be formed from a multilayer film, which is a stack of films made of different metals. A surface of the multilayer metal film is provided with plating having a higher strength, to further increase the mechanical strength.

The plating is effective in increasing the strength of a puncture device made of a metal, such as stainless steel, or a synthetic resin. The plating may be selected from, for example, bright chromium, velour chromium, bright nickel, velour nickel, bright tin-cobalt, velour tin-cobalt, bright gold, velour gold, trivalent chromium, bright Noburoi, velour Noburoi, rhodium plating, and platinum plating.

The above examples describe the channels, which are the holes formed in the basal portion in the thickness direction. However tha channels in the first and second examples may be grooves formed along the sides of the basal portion.

In addition to the case in which the drug is percutaneously administered by being guided from the second surface to the first surface of the basal portion through the channels formed in the basal portion, the above examples describe the case in which the drug is percutaneously administered through the cut by being guided to the blade portion through the channel formed in the basal portion and the through hole provided in the support portion of the bladed drug supply member or by being directly supplied to the cut from the opening end of the through hole provided in the support portion. However the blade portion may be coated with a drug in advance, and the drug may be percutaneously administered through the cut at the same time as the blade portion cuts the skin.

The above embodiments and examples describe that the basal portion has the discoid shape. The present invention is not limited to this. The basal portion may be a triangular or rectangular plate-shaped member.

The above embodiments and examples describe that the distal end portions of the blade portions have the gently curved shapes when viewed from the front. The present invention is not limited to this. The distal end portion of the blade portion may have a pointed shape when viewed from the front.

The above embodiments and examples describe that the blade portions have the shapes similar to the sides of the shells of a clam, when viewed from the side. The present invention is not limited to this. As shown in Fig. 10, a blade portion 46 may have a substantially isosceles triangle shape when viewed from the side. As shown in Fig. 11, a blade portion 48 may have a shape in which a thick proximal end is connected linearly to a tapered distal end, when viewed from the side.

In the first embodiment, the thicknesses of the support portions 38A and 38B of the bladed drug solution supply members 30A and 30B are not specifically limited as long as the through holes 32 are formed inside.

The blade portions 36 may be provided not only on the distal end portions 37 of the bladed drug solution supply members 30A and 30B but also on the entire outer edges of the bladed drug solution supply members 30A and 30B.

The first embodiment may be combined with the second example. For example, grooves 22 like those shown in Fig. 4 may be provided on the first and second surfaces of the bladed drug supply member. The through hole 32 may be provided with its opening inside the groove 22. In this case, the groove 22 may be provided from a height approximately 1/3 of the height of the bladed drug solution supply member 30A or 30B from the proximal end of the bladed drug solution supply member 30A or 30B. The angle of the distal end may be set to approximately 15° when viewed from the side. The groove 22 is not limited to a straight shape and may be angled at an angle of approximately 6°.

### [Reference Numerals]

- 10: puncture device
- 12: basal portion
- 14: puncture portion
- 16A to 16F, 36, 46, 48: blade portion
- 17, 28, 37: distal end portion
- 18: channel
- 20: widened portion (drug permeation portion)
- 22: groove (drug permeation portion)
- 24: teeth
- 26: proximal end portion
- 38A, 38B: support portion
- 30A, 30B: bladed drug supply member
- 32: through hole
- 34: opening end

## Claims

1. A puncture device (10) comprising:
a basal portion (12); and
a plurality of blade portions (36, 46, 48) disposed on the basal portion,
**characterized in that**
the blade portion has a convex shape when viewed from the front, and has a substantially elliptical shape or an oval shape when viewed from above, the proximal end of the blade portion has a certain thickness when viewed from the side, the blade portion tapers from the proximal end to the distal end when viewed from the side, a distal end portion (37) of the blade portion having a convex shape,
the basal portion includes a channel (18) guiding a drug to a first surface of the basal portion from a second surface of the basal portion, the blade portion being disposed on the first surface, and
the blade portion is fixed to the basal portion through a support portion (38A, 38B) integrally provided with the proximal end of the blade portion, and the support portion includes a through hole (32) communicating with the channel.

2. The puncture device according to claim 1, wherein a center portion of the basal portion protrudes toward the distal end of the blade portion.

3. The puncture device according to any one of claims 1 to 2, wherein the blade portion is formed from a synthetic resin, polylactic acid biodegradable polymer, or metal.

4. The puncture device according to claim 3, wherein a surface of the blade portion is provided with a metal film which reinforces a mechanical strength of the blade portion.

## Patentansprüche

1. Punktionsvorrichtung (10), umfassend:
einen Basisabschnitt (12); und
eine Vielzahl von Klingenabschnitten (36, 46, 48), die an dem Basisabschnitt angeordnet sind,
**dadurch gekennzeichnet, dass**
der Klingenabschnitt eine konvexe Form aufweist, wenn er von vorne betrachtet wird, und eine im Wesentlichen elliptische Form oder eine ovale Form aufweist, wenn er von oben betrachtet wird, das proximale Ende des Klingenabschnitts eine bestimmte Dicke aufweist, wenn er von der Seite betrachtet wird, der Klingenabschnitt sich von dem proximalen Ende zu dem distalen Ende hin verjüngt, wenn er von der Seite betrachtet wird, wobei ein distaler Endabschnitt (37) des Klingenabschnitts eine konvexe Form aufweist,
der Basalabschnitt einen Kanal (18) aufweist, der ein Medikament von einer zweiten Oberfläche des Basalabschnitts zu einer ersten Oberfläche des Basalabschnitts führt, wobei der Klingenabschnitt auf der ersten Oberfläche angeordnet ist, und
der Klingenabschnitt an dem Basalabschnitt durch einen Stützabschnitt (38A, 38B) befestigt ist, der einstückig mit dem proximalen Ende des Klingenabschnitts ausgebildet ist, und der Stützabschnitt ein Durchgangsloch (32) aufweist, das mit dem Kanal in Verbindung steht.

2. Punktionsvorrichtung nach Anspruch 1, wobei ein Mittelabschnitt des Basisabschnitts in Richtung des distalen Endes des Klingenabschnitts vorspringt.

3. Punktionsvorrichtung nach einem der Ansprüche 1 bis 2, wobei der Klingenabschnitt aus einem Kunstharz, einem biologisch abbaubaren Polymilchsäurepolymer oder einem Metall gebildet ist.

4. Punktionsvorrichtung nach Anspruch 3, wobei eine Oberfläche des Klingenabschnitts mit einer Metallfolie ausgerüstet ist, die eine mechanische Festigkeit des Klingenabschnitts verstärkt.

## Revendications

1. Dispositif de piqûre (10) comprenant :
une partie de base (12) ; et
une pluralité de parties de lame (36, 46, 48) disposées sur la partie de base, **caractérisé en ce que**
la partie de lame a une forme convexe lorsqu'elle est vue de devant, et a une forme sensiblement elliptique ou une forme ovale lorsqu'elle est vue du dessus, l'extrémité proximale de la partie de lame a une certaine épaisseur lorsqu'elle est vue de côté, la partie de lame s'effile de l'extrémité proximale jusqu'à l'extrémité distale lorsqu'elle est vue de côté, une partie (37) d'extrémité distale de la partie de lame ayant une forme convexe,
la partie de base inclut un canal (18) guidant un médicament jusqu'à une première surface de la partie de base depuis une deuxième surface de la partie de base, la partie de lame étant disposée sur la première surface, et
la partie de lame est fixée à la partie de base par l'intermédiaire d'une partie support (38A, 38B) prévue de façon intégrée avec l'extrémité proximale de la partie de lame, et la partie support inclut un trou traversant (32) communiquant avec le canal.

2. Dispositif de piqûre selon la revendication 1, dans lequel une partie centrale de la partie de base fait saillie vers l'extrémité distale de la partie de lame.

3. Dispositif de piqûre selon l'une quelconque des revendications 1 à 2, dans lequel la partie de lame est formée à partir d'une résine synthétique, d'un polymère de poly(acide lactique) biodégradable, ou d'un métal.

4. Dispositif de piqûre selon la revendication 3, dans lequel une surface de la partie de lame est prévue avec un film métallique qui renforce une résistance mécanique de la partie de lame.
